(19) Europäisches Patentamt
European Patent Office
Office européen des brevets

(11) Publication number: **0 204 394 B1**

# EUROPEAN PATENT SPECIFICATION

(45) Date of publication of patent specification: **26.06.91**   (51) Int. Cl.⁵: **A61B 5/02, A61B 5/0205**

(21) Application number: **86301304.1**

(22) Date of filing: **24.02.86**

(54) Apparatus for evaluating heart mechanical performance.

(30) Priority: **03.06.85 US 740524**

(43) Date of publication of application:
**10.12.86 Bulletin 86/50**

(45) Publication of the grant of the patent:
**26.06.91 Bulletin 91/26**

(84) Designated Contracting States:
**AT BE CH DE FR GB IT LI NL SE**

(56) References cited:
**EP-A- 0 046 570**      **DE-A- 2 648 889**
**DE-A- 2 736 377**      **DE-B- 2 845 889**
**US-A- 3 556 084**      **US-A- 3 776 221**
**US-A- 3 908 639**      **US-A- 3 924 612**

(73) Proprietor: **McIntyre, Kevin M.**
**160 Commonwealth Avenue**
**Boston Massachusetts 02116(US)**

(72) Inventor: **McIntyre, Kevin M.**
**160 Commonwealth Avenue**
**Boston Massachusetts 02116(US)**

(74) Representative: **Brunner, Michael John et al**
**GILL JENNINGS & EVERY 53-64 Chancery Lane**
**London WC2A 1HN(GB)**

## Description

The present invention relates in general to evaluating the mechanical performance of the heart and more particularly concerns novel apparatus for making this evaluation with relatively inexpensive apparatus that is relatively easy to operate by relatively unskilled personnel.

The present invention represents an improvement of the invention disclosed in US-A-3776221. That patent discloses establishing the time derivative of the systemic arterial pulse pressure at a control level in the subject patient. Then the patient performed a straining maneuver, such as a Valsalva maneuver, while recording the time derivative of the systemic arterial pulse pressure signal. Preferably, the systemic arterial pulse pressure, mean pressure, heart rate and left ventricular ejection time were also established and could be interpreted so that the presence or absence of impairment in the performance of the left ventricle could be detected.

It is an important object of this invention to provide improved apparatus and techniques for evaluating mechanical heart performance.

According to the present invention, apparatus for use in a method of evaluating the mechanical condition of a heart, comprising pressure applying means for applying pressure to the skin of a patient and for maintaining said pressure in a range from substantially just above diastolic to substantially half diastolic; pressure sensitive means adapted to be responsive to arterial pulsation for providing a pulse signal; and, means for detecting the change in the pulse signal during and after a heart straining maneuver relative to the pulse signal just before the heart straining maneuver; is characterised by: means defining a confined volume into which a patient can expire to develop expiration pressure therein; means for providing an expiration signal representative of expiration pressure in the confined volume; means for receiving the expiration pressure signal; and, means for providing from said expiration pressure signal an indication of the expiration pressure; the pressure applying means including the pressure sensitive means.

An electrical pulse signal representative of systemic arterial pulse pressure is produced. The pressure applying means may be an inflatable cuff. The preferred pressure is substantially diastolic, which seems to maximize sensitivity and repeatability. There is preferably a pressure source coupled to the pressure applying means for providing pressure to the latter.

Preferably, there is means for displaying a pulse signal characteristic of the electrical pulse signal, displaying an indication of the peak pressure on each pulse beat and an indication of the time interval between consecutive pulse beats. An expiration means for receiving expired air from a patient to be evaluated may comprise a confined volume with a small aperture for releasing the expired air and pressure sensing means coupled to the confined volume for providing an electrical air pressure signal representative of the pressure established in the confined volume by the expiring patient. Preferably, a display means provides an indication of the air pressure signal as a function of time.

Reliable results can be achieved with this system which is suitable for use in the office of an individual medical practitioner using noninvasive techniques with little discomfort to the patient being evaluated and whilst providing a reliable indication of mechanical heart pumping performance.

Using the apparatus of the invention, the transducing means is placed adjacent the skin, preferably a finger. Pressure is applied to the skin at least in part though the transducing means, preferably with an inflatable cuff surrounding the digit and transducing means. Preferably, there is a pressure source coupled to the cuff, such as a sphygmomanometer bulb coupled through a hose including a valve that may be closed when a desired pressure is reached, typically determined by observing a pressure indicator, to establish a base level of the pulse signal before subjecting the patient to a training maneuver. In this maneuver the patient then blows into the expiration means until the pressure indicating means indicates air pressure in the confined volume within a predetermined range, typically 30 to 50 mm of mercury (about 4 to 6.7 kPa), for a predetermined time interval, typically 20 seconds, during this straining maneuver, such as phases I and II of a Valsalva maneuver. The patient then breathes normally while the transduced pulse signals are recorded during phases I, III and IV of a Valsalva maneuver. The signals may be processed to provide an indication of one or more of the heart rate, time derivative of the pressure signal, integral of the pressure signal, peak pressure, mean pressure and diastolic pressure. One or more of a display of these signals for the four phases of the Valsalva maneuver may be observed to determine an evaluation of the mechanical performance of the heart.

One example of the apparatus according to the invention will now be described with reference to the accompanying drawing in which:

Figure 1 is a combined pictorial-block diagram illustrating the logical arrangement of the system;

Figure 2 is a graphical representation of a typical pulse signal just before during and following a Valsalva maneuver for a normal or "sinusoidal" response;

Figure 3 is a representation the same as Figure 2 except for an intermediate response with no overshoot;
Figure 4 is a representation similar to Figure 2 except representative of an abnormal or square-wave response;
Figure 5 is an exploded view of an example of a transducing assembly for pulse detecting;
Figure 6 is a plan view of a portion of a metal backed piezoelectric film;
FIG. 7 is a side view partially in section illustrating connections to the piezoelectric film; and
FIG. 8 shows a view of a CRT display for helping a patient control expiration pressure.

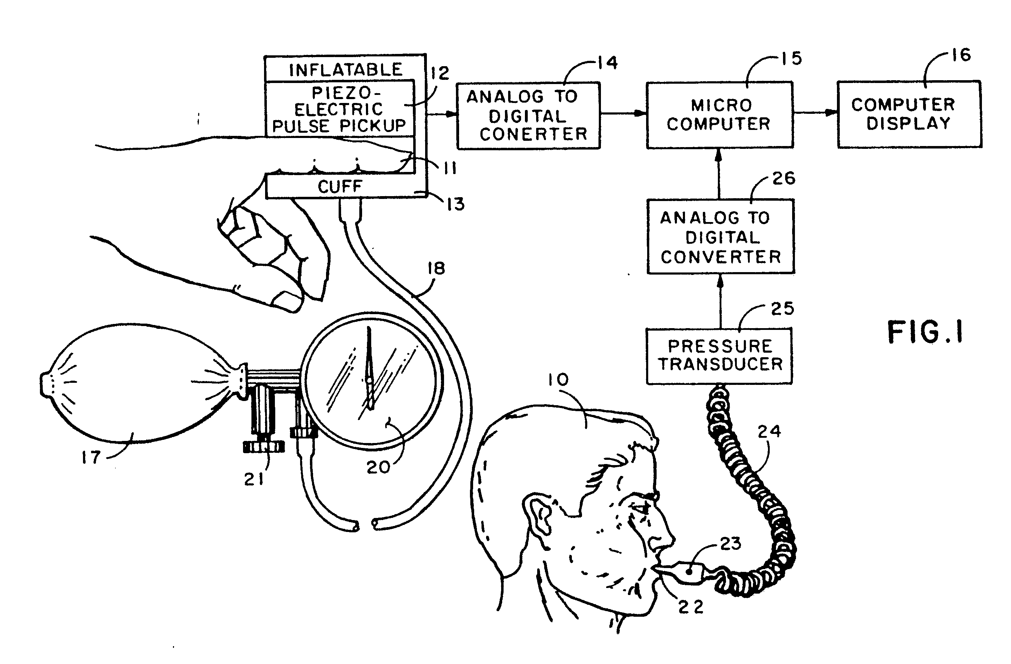

With reference now to the drawing and more particularly FIG. 1 thereof, there is shown a combined pictorial-block diagram illustrating the logical arrangement of a system according to the invention. A patient 10 to be evaluated places a finger 11 between piezoelectric pulse pickup 12 and inflatable cuff 13 to provide a pulse signal converted by analog to digital converter 14 to digital form that is processed by microcomputer 15 to provide on display 16 representation of the pulse sensed by pickup 12. Pressure source 17, typically a sphygmomanometer bulb connected to cuff 13 through hose 18 having a valve 21 for closing hose 18 when inflatable cuff 13 applies pressure at least in part through piezoelectric pulse pickup 12 to the skin of finger 11 surrounded by cuff 13. Pressure indicator 20 indicates the pressure, preferably substantially the diastolic pressure of patient 10. If the cuff pressure is significantly above diastolic pressure, the signal provided by pickup 12 may be small and may result in a less accurate diagnosis. If the cuff pressure is about diastolic pressure, the latter signal is maximized. The diastolic pressure for the patient may be determined by taking the patient's blood pressure in a conventional manner.

A mouthpiece 22, preferably replaceable, is coupled to hose 24 closed at the far end to define a confined volume and formed with an aperture 23 to release expired air. Pressure transducer 25 is coupled to hose 24 and provides a pressure signal converted by analog to digital converter 26 into digital form representative of the pressure in the confined volume. Microcomputer 15 processes the digital pressure signal to provide a representative signal on computer display 16 representative of the pressure in hose 24.

Having described the system arrangement, its mode of operation will be described. Patient 10 inserts finger 11 adjacent piezoelectric pulse pickup 12 in inflatable cuff 13. The operator then activates pressure source 17 to increase the pressure in cuff 13 until pressure indicator 20 indicates a pressure of about diastolic pressure. The valve 21 may be opened to reduce the pressure in inflatable cuff 13 to the desired pressure just before and throughout the maneuver. Patient 10 then blows into mouth piece 22 to create a pressure in hose 24 of magnitude indicated on computer display that is maintained for preferably 8 to 20 seconds between limits of 30-50 mm of mercury. This strained breathing creates the strain for phases I and II of the Valsalva maneuver. The patient then removes the mouthpiece and relaxes while the recovery and steady state phases of the maneuver follow.

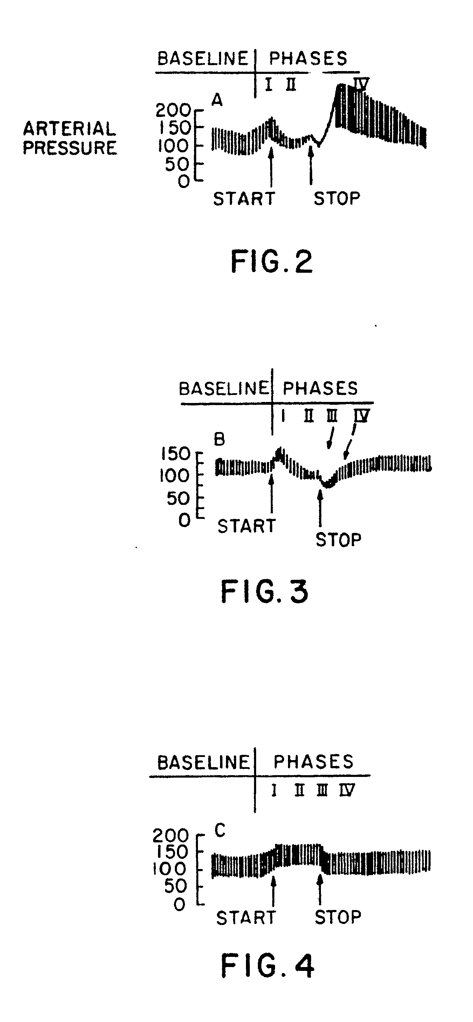

Referring to FIG. 2, there is shown a graphical representation of a pulse waveform just before and during the four phases of a Valsalva maneuver for a normal response showing the changes in arterial pressure relative to the pressure in the control interval just before straining as a function of time during a period of straining between the arrows marked start and stop. There is a rise in pressure on straining (phase I), a drop in pressure (phase II), fall below the baseline (phase III) and post straining rise (phase IV). The characteristics of the usual response of FIG. 2 are set forth in the following Table I relative to the baseline status. 0, ↑, ↓ and ↓↓ indicate no change, increase, decrease and large decrease respectively. An advantage of the invention is that measurements normalized to the prestrain interval for each patient during each test characterize mechanical heart performance.

## Table I

|  |  | Phase I | Phase II | Phase III | Phase IV |
|---|---|---|---|---|---|
| (1) | Ht. Rate | 0 or ↓ | ↑ | ↑ | ↓ |
| (2) | "dp/dt" | 0 | ↓ | ↓ | ↑ |
| (3) | ∫ pdt | 0 | ↓ | ↓↓ | ↑ |
| (4) | Impulse Amplitude | 0 | ↓ | ↓↓ | ↑ |
| (5) | Peak (systolic) Pressure | ↑ | ↓ | ↓ | ↑ |
| (6) | Mean Pressure | ↑ | ↑ 0 or ↓ | ↓ | ↑ |
| (7) | Diastolic Pressure or "Low" Pressure | ↑ | ↓ or 0 or ↑ | ↓ | ↑ |

Referring to FIG. 3, there is shown an intermediate abnormal response with no overshoot defined by the "systolic" level of pressure. The waveform of FIG. 3 relative to the baseline period has the characteristics set forth in Table II.

## Table II

|  |  | Phase I | Phase II | Phase III | Phase IV |
|---|---|---|---|---|---|
| (1) | Ht. Rate | 0 | 0 or ↑ | 0 or ↑ | 0 or ↑ |
| (2) | "dp/dt" | 0 | 0 or ↓ | 0 or ↓ | 0 |
| (3) | ∫ pdt | 0 | 0 or ↓ | ↓ | 0 |
| (4) | Impulse Amplitude | 0 | ↓ | ↓ | 0 |
| (5) | Peak (systolic) Pressure | ↑ | ↓ | ↓ | 0 |
| (6) | Mean Pressure | ↑ | 0 or ↓ | ↓ | 0 |
| (7) | Diastolic Pressure or "Low" Pressure | ↑ | 0 or ↓ | ↓ | 0 |

Referring to FIG. 4, there is shown a graphical representation of arterial pressure as a function of time for an abnormal response to a Valsalva maneuver. The characteristics of this response relative to the base period response is set forth in Table III.

4

Table III

| | | Phase I | Phase II | Phase III | Phase IV |
|---|---|---|---|---|---|
| (1) | Ht. Rate | 0 | 0 | 0 | 0 |
| (2) | "dp/dt" | 0 | 0 | 0 | 0 |
| (3) | ∫ pdt | 0 | 0 | 0 | 0 |
| (4) | Impulse Amplitude | 0 | 0 | 0 | 0 |
| (5) | Peak (systolic) Pressure | 0 | ↑ | ↑ | 0 |
| (6) | Mean Pressure | 0 | ↑ | ↑ | 0 |
| (7) | Diastolic Pressure or "Low" Pressure | 0 | ↑ | ↑ | 0 |

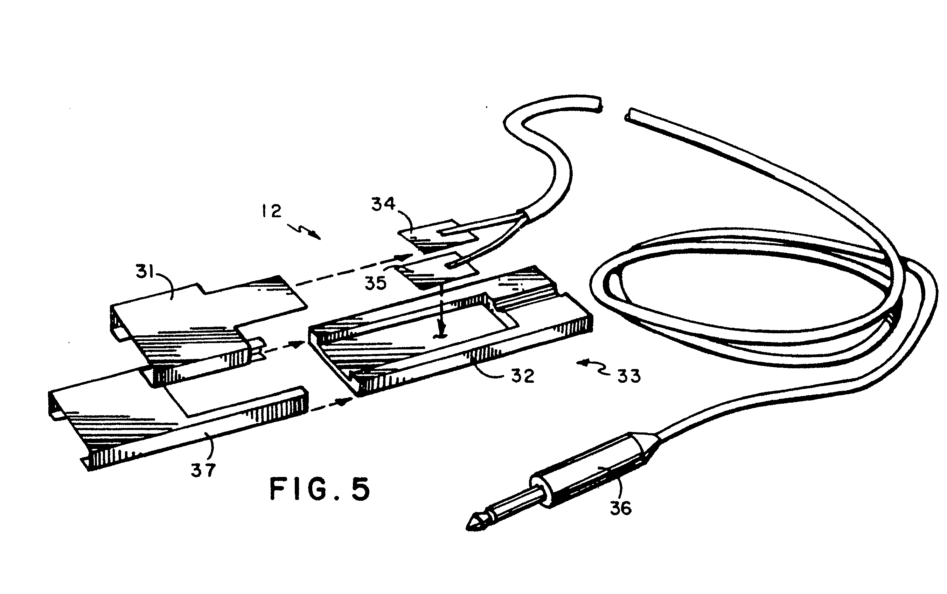

Referring to FIG. 5, there is shown an exploded view of an exemplary pulse pickup assembly 12 for use in the example. A piezoelectric film 31 is stretched across legs 32 of holder 33 and contacted on opposite sides by a pair of leads 34, 35 brought out to a miniature phono plug 36 that may plug into analog-to-digital converter 14 (FIG. 1). A around shield 37 completes the assembly. While FIG. 5 shows the pickup assembly of generally rectangular form, pickups of other shapes may be used, for example, pickups with a circular opening in the tip exposing a circular piezoelectic membrane surface. Membrane 31 is typically a commercially available polymer.

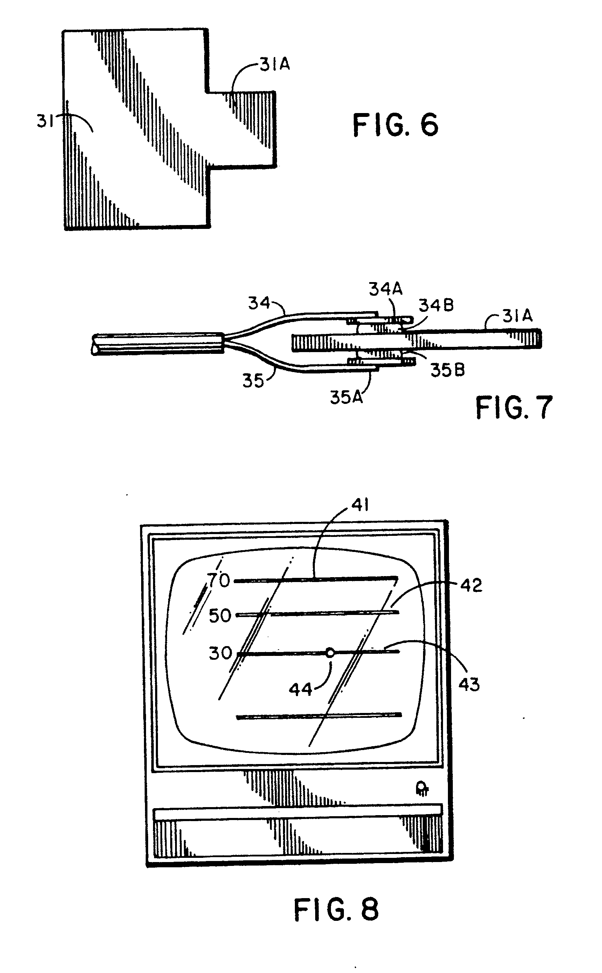

Referring to FIG. 6, there is shown a fragmentary plan view of the end portion of membrane 31 including stem 31A to which the contacts are attached. Referring to FIG. 7, there is shown a side fragmentary view illustrating the detail for connection to stem 31A. Leads 34 and 35 are soldered to metal buttons 34A and 35A, respectively, which are connected to opposite sides of tab 31A by dabs of conductive epoxy 34B and 35B, respectively.

Referring to FIG. 8, there is shown a view of a CRT display which may be used for allowing the patient to maintain a preferred pressure range during the test. Upper, intermediate and lower horizontal traces 41, 42 and 43, respectively, may represent pressures of 70, 50 and 30 mmHg, respectively. The patient blows hard enough on mouth piece 22 so as to maintain spot 44 between traces 42 and 43.

The blood pressure may be determined by the standard cuff-sphygmomanometer method or by an automatic system, such as the Doppler technique, or any other technique for providing an indication of blood pressure.

Specific components of the system are known in the art. The techniques for using a microcomputer to provide a representation of the peak pressure of each pulse and the pulse are well within the skill of a person having ordinary skill in the computer art and are not described in detail herein to avoid obscuring the principles of the invention. In a specific embodiment of the invention, microcomputer 15 was an Apple 2e with an associated picture tube display.

The Valsalva maneuver entails the abrupt development of an increase in intrathoracic pressure by forced expiration against a closed glottis. Such an increase in intrathoracic pressure results in a decrease in venous return to the heart, so that filling of the pumping chambers of the heart is normally decreased. Impairment of the filling of the left ventricle (the major pumping chamber of the heart) during this so-called "strain" phase results in a prompt fall in blood pressure in the normal individual, followed by an abrupt increase in pulse rate. At termination of "strain" phase in the normal individual, venous return to the heart is abruptly augmented, to a point which generally exceeds the original baseline rate of heart filling. The normal ventricle will increase its work to a level in excess of the "control" level in order to deal with the increase in filling (see FIG. 2). The result is that an "overshoot" occurs in which pulse pressure and blood pressure exceed their levels during the control or baseline level. The body senses this change and responds by reflexly slowing the heart via a sensing mechanism in the carotid arteries, the carotid baroreceptors. This normal response has several characteristics that distinguish it from the abnormal response of the patient with ventricular failure:

5

1) there is usually a fall in blood pressure during the "strain" phase;
2) there is an increase in heart rate toward the end of "strain" phase;
3) there is an "overshoot" shortly after release of the "strain" phase;
4) there is a slowing of heart rate in the course of the "overshoot"; and,
5) there is fairly prompt return to the baseline level of blood pressure, pulse pressure, and heart rate thereafter.

In the patient with cardiac failure, the physiological response to the Valsalva maneuver is quite different. One characteristic response is called the "square wave" response shown in FIG. 4 which involves an increase in blood pressure with the initiation of the strain phase and maintenance of the same blood pressure, pulse pressure and heart rate at this level throughout the "strain" phase. The "recovery" phase which follows the release of the Valsalva maneuver "strain" phase is characterized by a drop in the blood pressure to the baseline level, the absence of any "overshoot" and the absence of recovery period cardiac slowing.

In the example, sensitive analysis of these differences, either by the use of a derivative of the pressure signal from the pickup or by analysis of changes in systolic pressure, pulse pressure and heart rate at various phases, and an integral of that signal and a measure of the heart rate, facilitates using this known physiological response as a clinical indicator for cardiac status with an apparatus that is compact, relatively inexpensive and relatively easy to operate to produce reliable results. The present invention provides means that enable a practicing physician to detect in his office the presence of occult left ventricular (and/or right ventricular) failure as well as significant abnormalities of heart valves and constrictive and restrictive conditions which affect heart pumping function.

The invention is especially advantageous because the symptons of left ventricular failure are similar to those associated with other very common and other forms of heart disease conditions, some of which may be less threatening. For example, shortness of breath, a very common sympton of heart failure, may be caused by other conditions, e.g. shortness of breath is common among cigarette smokers. With increasing age and some progression of a disease, such as chronic lung disease, the ability to ascertain the presence of underlying heart failure as a contributing factor to a limiting symptom such as shortness of breath becomes increasingly difficult and important. The invention helps discriminate between heart failure and other processes which may produce physical symptoms similar to or identical to those caused by heart failure.

An important advantage of the invention is that it may be used to evaluate the results of treatment for heart conditions. And the ease of use, minimal discomfort to the patient and accessibility to all practitioners enhances health care.

## Claims

1. Apparatus for use in a method of evaluating the mechanical condition of a heart; the apparatus comprising:
   pressure applying means (13) for applying pressure to the skin of a patient and for maintaining said pressure in a range from substantially just above diastolic to substantially half diastolic; pressure sensitive means (12) adapted to be responsive to arterial pulsation for providing a pulse signal; and; means (14;15;16;22-26) for detecting the change in the pulse signal during and after a heart straining maneuver relative to the pulse signal just before the heart straining maneuver; characterised by:
   means (24) defining a confined volume into which a patient can expire to develop expiration pressure therein; means (25) for providing an expiration signal representative of expiration pressure in the confined volume (24); means (15) for receiving the expiration pressure signal; and, means (16) for providing from said expiration pressure signal an indication of the expiration pressure; the pressure applying means (13) including the pressure sensitive means (12).

2. Apparatus in accordance with claim 1, further comprising:
   container means carrying the pressure sensitive means (12) and inflatable means (13) for applying pressure to the patient's skin through means including the pressure sensitive means (12); and; means (17;20;21) for selectively inflating the inflatable means (12) to and maintaining the desired pressure just before, during and at least shortly after the heart straining maneuver.

3. Apparatus in accordance with claim 1 or claim 2, wherein the pressure applying means (13) comprises an inflatable cuff for applying pressure to the skin of a patient's digit.

6

4. Apparatus in accordance with any of claims 1 to 3, wherein the means for detecting the change in the pulse signal after the maneuver relative to the pulse signal before the maneuver includes means for detecting the change in rate and amplitude.

5. Apparatus in accordance with any of claims 1 to 4, wherein the means for detecting the change in the pulse signal after the maneuver relative to the pulse signal before the maneuver includes means for detecting changes in heart rate, the time derivative of a signal related to blood pressure, the time integral of the signal related to blood pressure, the impulse amplitude of the pulse signal, the peak systolic pressure represented by the pulse signal, the mean pressure represented by the pulse signal and the diastolic pressure represented by the pulse signal.

6. Apparatus in accordance with any of claims 1 to 5, further including means (16) for providing a display of the peak pressures represented by the sequence of pulses and the inverse of the time interval between consecutive pulses corresponding substantially to instantaneous heart rate.

**Revendications**

1. Appareil utilisable dans un procédé d'évaluation de l'état mécanique d'un coeur, l'appareil comprenant : un moyen d'application de pression (13) pour appliquer une pression à la peau d'un patient et pour maintenir ladite pression dans une gamme comprise entre une valeur sensiblement juste au-dessus de la pression diastolique et une valeur située sensiblement à la pression semi-diastolique, un moyen sensible à la pression (12) adapté pour être sensible à la pulsation artérielle pour fournir un signal de pouls, et un moyen (14, 15, 16, 22-26) pour détecter la variation du signal de pouls pendant et après un exercice d'effort cardiaque par rapport au signal de pouls juste avant l'exercice d'effort cardiaque, caractérisé par :
un moyen (24) définissant un volume confiné dans lequel un patient peut expirer pour y développer une pression d'expiration, un moyen (25) pour produire un signal d'expiration représentant la pression d'expiration dans le volume confiné (24), un moyen (15) pour recevoir le signal de pression d'expiration et un moyen (16) pour produire à partir dudit signal de pression d'expiration une indication de la pression d'expiration, le moyen d'application de pression (13) incluant le moyen sensible à la pression (12).

2. Appareil selon la revendication 1, comprenant en outre :
un récipient portant le moyen sensible à la pression (12) et un moyen gonflable (13) pour appliquer une pression à la peau du patient par l'intermédiaire d'un moyen incluant le moyen sensible à la pression (12), et un moyen (17, 20, 21) pour gonfler sélectivement le moyen gonflable (12) à la pression souhaitée et pour maintenir cette pression juste avant, pendant et au moins peu de temps après l'exercice d'effort cardiaque.

3. Appareil selon la revendication 1 ou la revendication 2, dans lequel le moyen d'application de pression (13) comprend un manchon gonflable pour appliquer une pression à la peau d'un doigt du patient.

4. Appareil selon l'une quelconque des revendications 1 à 3, dans lequel le moyen pour détecter la variation du signal de pouls après l'exercice par rapport au signal de pouls avant l'exercice comprend un moyen pour détecter la variation de rythme et d'amplitude.

5. Appareil selon l'une quelconque des revendications 1 à 4, dans lequel le moyen pour détecter la variation du signal de pouls après l'exercice par rapport au signal de pouls avant l'exercice comprend un moyen pour détecter les variations du rythme cardiaque, la dérivée par rapport au temps d'un signal correspondant à la pression sanguine, l'intrégale par rapport au temps du signal correspondant à la pression sanguine, l'amplitude d'impulsion du signal de pouls, le pic de pression systolique représenté par le signal de pouls, la pression moyenne représentée par le signal de pouls et la pression diastolique représentée par le signal de pouls.

6. Appareil selon l'une quelconque des revendications 1 à 5, comprenant en outre un moyen (16) pour produire un affichage des pics de pression représentés par la suite des pulsations et l'inverse des intervalles de temps entre les pulsations consécutives correspondant sensiblement au rythme cardiaque instantané.

EP 0 204 394 B1

**Ansprüche**

1. Vorrichtung zur Verwendung in einem Verfahren zur Beurteilung des mechanischen Zustandes des Herzes, mit:
Druckaufbringmitteln (13) zum Aufbringen eines Druckes auf die Haut des Patienten und zum Aufrechterhalten dieses Druckes in einem Bereich von etwa gerade über dem diastolischen Druck und bis zu etwa dem halben diastolischen Druck; druckempfindlichen Mitteln (12), die auf den arteriellen Pulsschlag ansprechen können, um ein Pulssignal zur Verfügung zu stellen; und Mitteln (14, 15, 16, 22 - 26) zum Erfassen einer Veränderung des Pulssignales während und nach einer herzbeanspruchenden Tätigkeit im Verhältnis zum Pulssignal genau vor der herzbeanspruchenden Tätigkeit; gekennzeichnet durch:
Mittel (24) zum Definieren eines begrenzten Volumens, in das der Patient ausatmen kann, so daß sich darin der Expirationsdruck aufbaut; Mittel (25) zum Erzeugen eines Expirationssignals, das den Expirationsdruck im begrenzten Volumen (24) darstellt; Mittel (15) zum Empfang des Expirationsdrucksignals; und Mittel (16) zum Erzeugen einer Anzeige des Expirationsdruckes aus diesem Expirationsdrucksignal; wobei die Druckaufbringmittel (13) die druckempfindlichen Mittel (12) beinhalten.

2. Vorrichtung nach Anspruch 1, weiters mit:
Gehäusemitteln, welche die druckempfindlichen Mittel (12) und die aufpumpbaren Mittel (13) tragen, um über Mittel, welche die druckempfindlichen Mittel (12) beinhalten, Druck auf die Haut des Patienten aufzubringen; und Mitteln (17, 20, 21) zum selektiven Aufpumpen der aufpumpbaren Mittel (12) auf den gewünschten Druck und zum selektiven Aufrechterhalten dieses Druckes genau vor, während und zumindest kurz nach der herzbeanspruchenden Tätigkeit.

3. Vorrichtung nach Anspruch 1 oder 2, bei welcher die Druckaufbringmittel (13) eine aufpumpbare Manschette zum Aufbringen von Druck auf die Haut eines Fingers des Patienten aufweisen.

4. Vorrichtung nach einem der Ansprüche 1 bis 3, bei welcher die Mittel zum Erfassen einer Veränderung des Pulssignales nach der Tätigkeit im Verhältnis zum Pulssignal vor der Tätigkeit Mittel zum Erfassen einer Veränderung der Frequenz und der Amplitude beinhalten.

5. Vorrichtung nach einem der Ansprüche 1 bis 4, bei welcher die Mittel zum Erfassen einer Veränderung des Pulssignales nach der Tätigkeit im Verhältnis zum Pulssignal vor der Tätigkeit Mittel zum Erfassen von Veränderungen der Herzfrequenz, der Zeitabgeleiteten eines mit dem Blutdruck zusammenhängenden Signales, der Zeitintegrierten dieses mit dem Blutdruck zusammenhängenden Signales, der Impulsamplitude des Pulssignales, des durch das Pulssignal dargestellten Spitzenwertes des systolischen Druckes, des durch das Pulssignal dargestellten mittleren Druckes und des durch das Pulssignal dargestellten diastolischen Druckes beinhalten.

6. Vorrichtung nach einem der Ansprüche 1 bis 5, weiters mit Mitteln (16) zum Erzeugen einer Anzeige der Druck-Spitzenwerte, die durch die Abfolge von Pulsschlägen dargestellt werden, und der Inversen des Zeitintervalls zwischen aufeinanderfolgenden Pulsschlägen, die im wesentlichen der momentanen Herzfrequenz entspricht.

8

FIG.1

FIG.2

FIG.3

FIG.4

FIG.5

EP 0 204 394 B1

FIG. 6

FIG. 7

FIG. 8